**Europäisches Patentamt**

**European Patent Office**

**Office européen des brevets**

(19)

(11) Veröffentlichungsnummer: **0 092 022**
**B1**

(12) ## EUROPÄISCHE PATENTSCHRIFT

(45) Veröffentlichungstag der Patentschrift:
07.01.87

(51) Int. Cl.⁴: **G 01 N 33/18**

(21) Anmeldenummer: **83100821.4**

(22) Anmeldetag: **28.01.83**

(54) **Verfahren und Vorrichtung zum Eichen von Sensoren.**

(30) Priorität: **08.04.82 DE 3213241**

(43) Veröffentlichungstag der Anmeldung:
**26.10.83 Patentblatt 83/43**

(45) Bekanntmachung des Hinweises auf die Patenterteilung:
**07.01.87 Patentblatt 87/2**

(84) Benannte Vertragsstaaten:
**AT CH DE FR GB IT LI NL**

(56) Entgegenhaltungen:
DE - A - 1 573 198
DE - A - 2 244 643
DE - C - 642 661
DE - C - 1 013 447
GB - A - 1 026 071
US - A - 3 926 561

**Die Akte enthält technische Angaben, die nach dem Eingang der Anmeldung eingereicht wurden und die nicht in dieser Patentschrift enthalten sind.**

(73) Patentinhaber: **WTW-Wissenschaftlich-Technische -Werkstätten GmbH, Trifthofstrasse 57a, D-8120 Weilheim (DE)**

(72) Erfinder: **Schuler, Peter, Dr., Schmautzer-Weg 13, D-8084 Inning (DE)**
Erfinder: **Herrnsdorf, Johannes, Am Anger 36, D-8126 Hohenpeissenberg (DE)**
Erfinder: **Talmer, Peter, Hörnlestrasse 14, D-8120 Weilheim (DE)**

(74) Vertreter: **Zipse + Habersack, Kemnatenstrasse 49, D-8000 München 19 (DE)**

## Beschreibung

Die Erfindung betrifft ein Verfahren und eine Vorrichtung zum Eichen von Sensoren gemäss dem Oberbegriff des Anspruchs 1 bzw. 7.

Zur Bestimmung des Sauerstoffgehalts von Wasser, Flüssigkeiten und Gasen setzt sich neben den klassischen Analyseverfahren wie z.B. der Titration nach Winkler im steigenden Masse der Einsatz elektrochemischer Sensoren durch, die im allgemeinen einfach zu handhaben sind. Dabei wird durch eine chemische Reaktion, wie z.B. Reduktion oder Oxidation, deren Intensität vom Sauerstoffpartialdruck abhängt, eine elektrische Grösse wie Strom oder Spannung erzeugt oder verändert, die dann messtechnisch bearbeitet werden kann. Als Beispiel eines solchen elektrochemischen Sensors sei die Clarkzelle erwähnt, die nach dem polarographischen Prinzip arbeitet.

Eine Umrechnungsmöglichkeit von Partialdruck auf die oft interessierende Massenkonzentration bietet das Henry-Dalton'sche Partialdruckgesetz, wobei eine Temperaturabhängigkeit messtechnisch zu berücksichtigen ist.

Auch andere Verfahren wie das galvanische Prinzip nach Mackereth, siehe Dick, Pittwell, Dissolved Oxygen in Natural and Waste Waters 1979 Version (1980) Her Majesty's Stationary Office, oder die Ross-Zelle siehe DE-OS 2 744 771, verarbeiten einen Strom, der dem Sauerstoffpartialdruck proportional ist. Prinzipiell sind auch Möglichkeiten über eine Potentialmessung oder aber über resultierende Grössen gegeben.

Alle Methoden nutzen eine Korrelation zwischen Sauerstoffpartialdruck und einer elektrischen Grösse. Das Problem der Zuordnung wird durch eine Eichung, in der Regel eine Zweipunkteichung gelöst.

Den einen Eichpunkt stellt eine sauerstofffreie Lösung z.B. Natriumsulfitlösung, womit $O_2$-unabhängige, systembedingte Restströme kompensiert werden. Der zweite Eichpunkt gibt den Bezug zu einer bekannten Konzentration bzw. zu einem bekannten Partialdruck.

So kann man die Sauerstoffkonzentration einer Lösung durch eine Titration nach Winkler bestimmen und dieser den zugehörigen elektrischen Wert zuordnen. Doch sind diese chemischen Verfahren recht aufwendig. Es bedarf spezieller Chemikalien und Geräte. Eine sorgfältige Eichung ist nur unter Laborbedingungen möglich.

Eine wesentliche Vereinfachung stellt die Eichung in luftgesättigtem Wasser dar. Man geht dann davon aus, dass der Sauerstoffpartialdruck in luftgesättigtem Wasser dem in wasserdampfgesättigter Luft entspricht, wobei sein Anteil am Gesamtdruck trockener Luft mit atmosphärischer Zusammensetzung bekanntlich 20,95 Vol Prozent ausmacht. Diesem kann man nun den Sondenstrom in luftgesättigtem Wasser zuordnen. Eine Umrechnung oder Eichung auf Massenkonzentrationen erfolgt über Sättigungstabellen, die die $O_2$-Konzentration bei Luftsättigung in Wasser angeben.

Auch dieses Eichverfahren beinhaltet Nachteile. So ist die Frage, wann Wasser als luftgesättigt angesehen werden kann, dem individuellen Massstab und der Sorgfalt des Anwenders überlassen. Zudem bedarf es auch hier eines gewissen Aufwandes. Man benötigt ein Gefäss mit Wasser und eine Vorrichtung, um dieses mit Luft zu sättigen. Weiterhin muss die Eichprobe gerührt werden, um für eine ausreichende Strömung am Sensor zu sorgen, damit der $O_2$-Partialdruck an der Membran mit dem in der Eichprobe stets vollkommen identisch ist, und Zehr-Effekte vermieden werden. In einem Labor mag es gelingen, mit diesem Verfahren eine Eichung vorzunehmen. Für den Feldeinsatz ist es jedoch nicht praktikabel.

Es ist daher naheliegend, unmittelbar an Luft zu eichen, wobei die besonderen Vorteile eines solchen Verfahrens auf der Hand liegen. Die Eichung an Luft kann mit geringem gerätetechnischem Aufwand durchgeführt werden. Allerdings sind auch hier einige Fehlerquellen zu beachten.

So ist zunächst einmal der Einfluss des Wasserdampfpartialdrucks zu berücksichtigen. Für den Sauerstoffpartialdruck ist demnach anzusetzen:

$$p_{O_2} = (p - p_{H_2O}) \times 0{,}2095$$

mit
$p$ = Gesamtluftdruck
$p_{H_2O}$ = Wasserdampfpartialdruck
$p_{O_2}$ = Sauerstoffpartialdruck

Um genaue Eichbedingungen zu erreichen, muss also der Wasserdampfpartialdruck und damit die relative Luftfeuchtigkeit bekannt sein. Sinnvollerweise wird man eine Eichung bei Wasserdampfsättigung anstreben.

In einigen Druckschriften, wie z.B. in M.L. Hitchman, Measurement of Dissolved Oxygen, John Wiley & Sons, Inc. and Orbisphere Laboratories Corporation, Genf, York (Maine) 1978, S. 90 beschrieben, wird empfohlen, in einem Abstand von weniger als 1 cm über einer Wasserfläche zu eichen, da dort Wasserdampfsättigung vorliege. Dieser Annahme gegenüber ist eine gewisse Skepsis angebracht, da keinerlei experimentelle Rahmenbedingungen festgelegt sind. Tatsächlich zeigt das Experiment, dass das Vorhandensein einer Wasserdampfsättigung über einem mit Wasser gefüllten Becherglas mit Recht anzuzweifeln ist. Bei einer grösseren Wasserfläche im Freien können undefinierte Luftströmungen die Eichung beeinflussen.

Einen Ausweg stellt hier der Gebrauch eines Eichgefässes dar, in das Wasser gefüllt wird, über dem dann der Sensor in wasserdampfgesättigter Luft geeicht wird. Zwar werden durch diese Methode externe Einflüsse vermieden, doch es ergibt sich auch hier ein entscheidender Nachteil. Bereits nach kurzer Zeit beschlägt die Membran mit Wassertropfen, was zu einer Verminderung des Elektrodensignals führt. Der Eichpunkt ist damit instabil und nicht exakt definiert.

Es ist Aufgabe der vorliegenden Erfindung, ein Verfahren zur Eichung von Sensoren zur Verfügung zu stellen, das für den Anwender ohne grossen gerätetechnischen Aufwand nutzbar ist und inbesondere für einen definierten und reproduzierbaren Wasserdampfpartialdruck in der Eichatmosphäre sorgt und eine Abscheidung von Wassertropfen an der Membran verhindert. Mit der Erfindung soll auch eine Vorrichtung zur Durchführung des Verfahrens geschaffen werden.

Die gestellte Aufgabe wird gelöst durch ein Verfahren gemäss Anspruch 1 bzw. 4 sowie durch eine Vorrichtung gemäss Anspruch 7 bzw. 17. Weiterbildungen der Erfindung sind in den abhängigen Ansprüchen beschrieben.

Eine Wassertropfenbildung an der Membran tritt erst dann auf, wenn Sättigungs- oder Übersättigungsverhältnisse vorliegen. Hier setzt die erfindungsgemässe Lösung an, indem sie im Untersättigungsbereich definierte und reproduzierbare Wasserdampfpartialdruckverhältnisse schafft, wobei eine Wasserdampfkonzentration in unmittelbarer Näherung an die Sättigung bevorzugt wird.

Überraschend einfach erreicht man diese Bedingungen, wenn man den zu eichenden Sensor in das zu messende Partialgas bringt, in dem aufgrund eines Konzentrationsgefälles von Sättigung zu Untersättigung ein Wasserdampfstrom fliesst. Legt man den Standort der Elektrode mit Hilfe eines Eichgefässes fest, so sind definierte und reproduzierbare Eichverhältnisse gewährleistet.

Einen solchen Wasserdampfstrom kann man ganz einfach dadurch erzeugen, dass man ein Eichgefäss mit mehreren Kammern benutzt, wobei die erste zum Teil mit Wasser gefüllt und über eine Bohrung mit einer zweiten «trockenen», dem Eichraum, verbunden ist. Dieser Eichraum ist seinerseits mit Löchern zu einer dritten Kammer, im bevorzugten Fall zur Umgebungsluft hin geöffnet. Auf diese Weise entsteht in dem Eichraum ein Wasserdampfstrom, der an der Messstelle für definierte und reproduzierbare Wasserdampfpartialdruckverhältnisse sorgt.

Anstelle einer zum Teil mit Wasser gefüllten Kammer kann auch ein beliebiges anderes Wasserdampfreservoir benutzt werden, wie beispielsweise ein saugfähiges, wassergetränktes Material wie ein Schwamm.

Eine andere Möglichkeit, im Untersättigungsbereich definierte und reproduzierbare Wasserdampfpartialdruckverhältnisse an der Messstelle zu schaffen, ohne dass es zur Wassertropfenbildung an der Membran kommt, besteht darin, den Sensor auf einer gegenüber dem wasserdampfgesättigten Partialgas erhöhten Temperatur zu halten. Dies kann auf zweierlei Weise erfolgen, indem entweder der Sensor nahe der Messstelle, d.h. der Membran, beheizt wird oder der Eichraum gekühlt wird. Eine Kühlung des Eichraums kann in einfacher Weise dadurch erfolgen, dass man an der Aussenwand des Eichraums eine Flüssigkeit wie Wasser oder eine andere leicht flüchtige Flüssigkeit verdunsten lässt.

Die Erfindung wird nachfolgend in bevorzugten Ausführungsformen anhand beigefügter Zeichnungen näher erläutert. Es zeigen

Fig. 1 ein Funktionsschema

Fig. 2 eine erste bevorzugte, labormässige Ausführungsform, und

Fig. 3 eine zweite bevorzugte Ausführungsform, bei der ein Bolzenverschluss das Eindringen von Wasser in den Eichraum verhindert, wenn das Eichgefäss nicht zur Eichung benutzt wird.

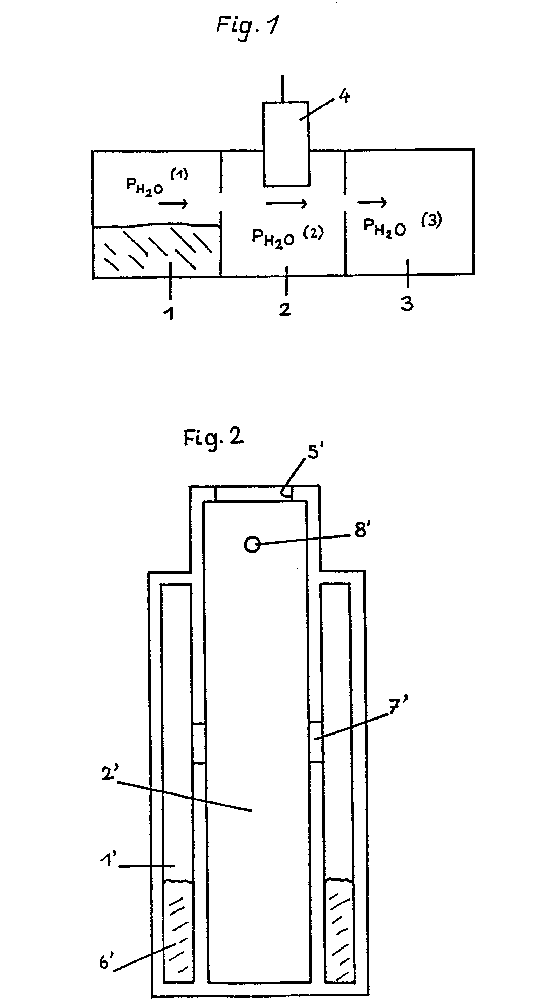

Das Funktionsprinzip, wie es die Fig. 1 erläutert, beruht auf einem Wasserdampfpartialdruckgefälle

$$p_{H_2O}(1) > p_{H_2O}(2) > p_{H_2O}(3)$$

in einem Dreikammersystem 1–3. In der teilweise mit Wasser gefüllten Kammer 1, dem Wasserdampfreservoir, herrscht im wesentlichen Wasserdampfpartialdrucksättigung, in Kammer 3, dem Ausweichreservoir, Wasserdampfpartialdruckuntersättigung vor. Infolgedessen wird ein Wasserdampfstrom von Kammer 1 nach Kammer 3 vorliegen. Der Sensor 4 wird in Kammer 2, dem Eichraum, bei einem Wasserdampfpartialdruck $p_{H_2O}(2)$ geeicht.

Eine modifizierte Anordnung in Fig. 2 zeigt eine zylindrische Symmetrie. Die dritte Kammer, das Aus-weichreservoir, wird durch die Umgebungsluft gebildet. Die mittlere Kammer 2' nimmt als Eichraum durch eine obere Öffnung 5' den (nicht dargestellten) Sensor dicht auf und ist von einem zylindrischen Rohr umgeben. Dieses wird teilweise mit Wasser 6' gefüllt und dient als Wasserdampfreservoir 1'. Durch die Bohrungen 7' und 8' fliesst ein Wasserdampfstrom vom Wasserdampfreservoir 1' am Sensor vorbei zur Umgebungsluft.

Eine Messung der Luftfeuchtigkeit in dem Eichraum 2' nach dem psychrometrischen Verfahren führt zu dem Ergebnis, dass die Luftfeuchtigkeit mit der Temperatur steigt und sich der Sättigung infinitesimal nähert, während in einem abgeschlossenen Gefäss sich stets nach kurzer Zeit 100% Luftfeuchtigkeit einstellt, was, wie bereits erwähnt, zu einer Wassertropfenbildung an der Sensormembran führt.

Gestalt und Lage der Luftfeuchtigkeitskurve in dem Eichraum ist für das Gefäss charakteristisch und reproduzierbar, so dass für die Eichung ein gerätespezifischer Korrekturfaktor festgelegt werden kann. Der Kurvenverlauf wird durch die Abmessungen des Eichgefässes und der Verbindungsbohrungen bestimmt. So können zu grosse Bohrungen zu Einflüssen aus der Umgebungsluft führen und die Reproduzierbarkeit der Eichung beeinträchtigen. Aufgrund experimenteller Erfahrung sollte ein Verhältnis von Durchtrittsfläche in mm² : Eichraumvolumen in mm³ = 1:300 nicht unterschritten werden. Bevorzugt werden Verhältnisse wie 1:1200 und grösser. Eine höhere Feuchtigkeit in dem Eichraum wird dann erreicht, wenn die Bohrungen zwischen Wasserdampfreservoir und Eichraum grösser sind als die zur Umgebungsluft.

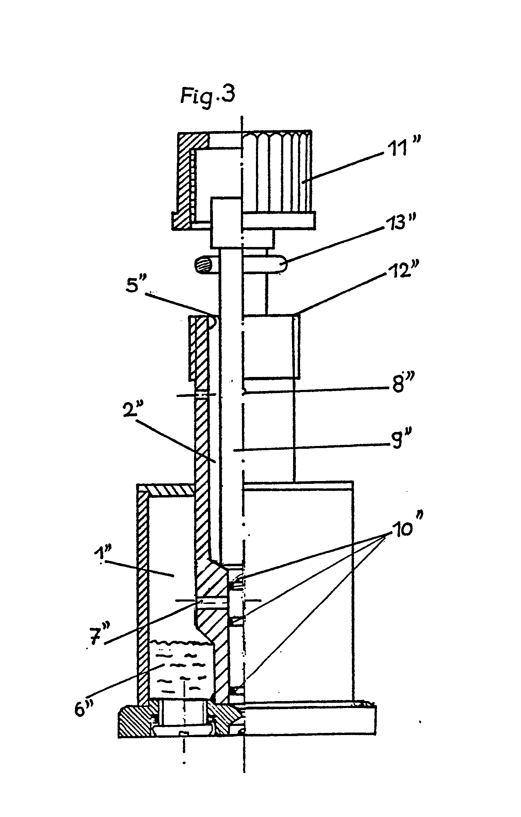

Ein weiteres Ausführungsbeispiel mit prinzipiell gleichem Aufbau wie in Fig. 2 gibt Fig. 3 wieder. In Eichpausen kann hier der Eichraum 2″ mit einem Bolzen 9″ mit O-Ringdichtungen 10″ von dem Wasserdampfreservoir 1″ dicht abgeschlossen werden, so dass kein flüssiges Wasser in den Eichraum eindringen kann, das bei späterer Benützung Wasserdampfsättigung im Eichraum bewirken könnte. Die Verbindungsbohrungen 7″ münden oberhalb der Oberfläche des Wassers 6″ im Wasserdampfreservoir 1″. Damit eventuell in den Eichraum eindringendes Wasser abfliessen kann, enthält der Eichraum in seinem Boden eine kleine Abflussbohrung, durch die das Wasser durch den Bolzen 9″ hinausgedrückt wird. Die Abflussbohrung ist klein und zudem während des Eichvorganges durch eine Unterlage abgedeckt, so dass sie keinen schädlichen Einfluss auf den Eichvorgang hat. Eine Schraubkappe 11″ klemmt zwischen sich und der Oberseite 12″ des Eichgefässes einen O-Ring 13″ ein, so dass dieser sich dichtend gegen die Mantelfläche eines in die Öffnung 5″ eingesetzten Sensors legt. Die Verbindungsbohrung des Eichraums 2″ zur Umgebungsluft ist mit 8″ bezeichnet.

Beim Eichvorgang wird der Bolzen entfernt und der (nicht dargestellte) Sensor dicht eingesetzt. Der Wasserdampfpartialdruckausgleich erfolgt erfahrungsgemäss innerhalb von 20 Sekunden, d.h. dass gemäss der erfindungsgemässen Lösung innerhalb von 20 Sekunden stabile Eichbedingungen geschaffen werden, während im abgeschlossenen System sehr schnell Instabilitäten auftreten.

Anstelle eines Abschlusses des Eichraumes vom Wasserdampfreservoir in Eichpausen mittels eines Schliessbolzens kann die Verbindung auch ständig durch ein poröses, wasserdampfdurchlässiges, aber für Wasser undurchlässiges Diaphragma abgedeckt werden, wodurch sich eine Vereinfachung in der Handhabung ergibt.

Das Eichgefäss sollte soweit thermisch isoliert sein, damit bei Abkühlung z.B. im Feldversuch genügend Zeit für einen Wasserdampfausgleich zur Umgebung verbleibt und der Taupunkt im Eichraum nie erreicht wird.

Sehr aufschlussreich war ein Experiment, in dem die Eichung in luftgesättigtem Wasser und im Eichgefäss verglichen wurden. Zunächst einmal liegt das Signal im Eichgefäss um 1...2% über dem in luftgesättigtem Wasser, was auf sensor- und gefässspezifische Eigenschaften zurückzuführen ist und bei der Eichung berücksichtigt werden kann. Das Signal in dem Eichraum weist mit einem Variationskoeffizienten <0,4% eine bessere Reproduzierbarkeit gegenüber luftgesättigtem Wasser mit einem Variationskoeffizienten von ca. 1,4% auf.

Mit dem vorliegenden Eichverfahren wird also dem Anwender eine Möglichkeit gegeben, sicher und reproduzierbar zu eichen, wobei die besonderen Vorteile der Lufteichung hinsichtlich ihrer Praktikabilität genutzt werden. Auch wenn die Eichung in Verbindung mit einer Sauerstoffpartialdruckmessung beschrieben wurde, versteht es sich dennoch, dass das erfindungsgemässe Verfahren sich auch zur Eichung von Sensoren eignet, die andere Gaspartialdrücke, wie z.B. von $CO_2$-Gas messen.

## Patentansprüche

1. Verfahren zum Eichen von Sensoren, die einen Gaspartialdruck und insbesondere einen Sauerstoffpartialdruck messen, bei dem der Sensor dem zu messenden Partialgas mit bekanntem Partialdruck und mit einem Wasserdampfgehalt, der aufgrund eines Partialdruckgefälles zwischen wasserdampfgesättigtem und untersättigtem Gas an der Sättigungsgrenze liegt, ausgesetzt und das Sensorsignal gemessen wird, dadurch gekennzeichnet, dass in einem Eichgefäss ein definierter Wasserdampfstrom von wasserdampfgesättigtem zu untersättigtem Gas erzeugt und der Standort der Messstelle innerhalb dieses Wasserdampfstroms so festgelegt wird, dass der Wasserdampfpartialdruck an der Messstelle sich der Wasserdampfsättigung nähert, diese aber nicht erreicht oder überschreitet.

2. Verfahren nach Anspruch 1, dadurch gekennzeichnet, dass der Wasserdampfstrom über enge Verbindungen von einem Wasserdampfreservoir zur Messstelle und von dieser zum untersättigten Gas geleitet wird, wobei die Verbindung von der Messstelle zum Wasserdampfreservoir grösser ist als die Verbindung der Messstelle zum untersättigten Gas.

3. Verfahren nach Anspruch 1 oder 2, dadurch gekennzeichnet, dass das untersättigte Gas Umgebungsluft ist.

4. Verfahren zum Eichen von Sensoren, die einen Gaspartialdruck und insbesondere einen Sauerstoffpartialdruck messen, bei dem der Sensor dem zu messenden Partialgas mit bekanntem Partialdruck und mit einem bestimmten Wasserdampfgehalt ausgesetzt und das Sensorsignal gemessen wird, dadurch gekennzeichnet, dass in dem zu messenden Partialgas Wasserdampfsättigung eingestellt und der Sensor auf einer gegenüber dem Partialgas erhöhten Temperatur gehalten wird.

5. Verfahren nach Anspruch 4, dadurch gekennzeichnet, dass der Sensor beheizt wird.

6. Verfahren nach Anspruch 4, dadurch gekennzeichnet, dass das Partialgas durch Verdunstung auf einem gegenüber dem Sensor niedrigeren Temperaturniveau gehalten wird.

7. Vorrichtung zur Durchführung des Verfahrens nach Anspruch 1, gekennzeichnet durch einen Eichraum (2, 2′, 2″), der einerseits mit einem Wasserdampfreservoir (1, 1′, 1″), das wasserdampfgesättigtes Partialgas enthält, und andererseits mit einem Ausweichreservoir (3), das nicht wasserdampfgesättigtes Partialgas enthält, verbunden ist.

8. Vorrichtung nach Anspruch 7, dadurch gekennzeichnet, dass die Verbindung des Eichraums (2',2") zu dem Wasserdampfreservoir (1',1") grösser ist als zu dem Ausweichreservoir.

9. Vorrichtung nach Anspruch 7 oder 8, dadurch gekennzeichnet, dass das Wasserdampfreservoir (1',1") eine teilweise mit Wasser (6',6") gefüllte Kammer ist und das Ausweichreservoir, das nicht wasserdampfgesättigtes Partialgas enthält, von der Umgebungsluft gebildet ist.

10. Vorrichtung nach Anspruch 9, dadurch gekennzeichnet, dass der Eichraum (2',2") und das Wasserdampfreservoir (1',1") durch zwei unten und oben abgeschlossene, konzentrisch ineinander gesteckte Rohre gebildet sind, wobei das innere Rohr den Eichraum bildet und eine Öffnung (5',5") zum dichten Einführen des zu eichenden Sensors aufweist, und dass die Verbindungen des Eichraums zum Wasserdampfreservoir einerseits und zur Umgebungsluft andererseits durch Bohrungen (7',7",8',8") geeigneten Durchmessers bewerkstelligt sind.

11. Vorrichtung nach Anspruch 7 oder 8, dadurch gekennzeichnet, dass das Wasserdampfreservoir durch ein saugfähiges, wassergetränktes Material gebildet ist.

12. Vorrichtung nach einem der Ansprüche 7 bis 11, dadurch gekennzeichnet, dass das Verhältnis der Durchtrittsquerschnitte in mm² der Verbindungen (7',7",8',8") des Eichraums (2',2") zum Wasserdampfreservoir (1',1") und zum Ausweichreservoir zu dem Volumen des Eichraums in mm³ 1:300 nicht unterschreitet.

13. Vorrichtung nach Anspruch 12, dadurch gekennzeichnet, dass dieses Verhältnis 1:1200 oder grösser ist.

14. Vorrichtung nach einem der Ansprüche 7 bis 13, dadurch gekennzeichnet, dass die Verbindung des Eichraums (2") zum Wasserdampfreservoir (1") in den Eichpausen verschliessbar ist.

15. Vorrichtung nach einem der Ansprüche 7 bis 14, dadurch gekennzeichnet, dass die Verbindung des Eichraums zum Wasserdampfreservoir durch ein poröses, wasserdampfdurchlässiges, aber für Wasser undurchlässiges Diaphragma abgedeckt ist.

16. Vorrichtung nach einem der Ansprüche 7 bis 15, dadurch gekennzeichnet, dass der Eichraum (2, 2',2") ausreichend thermisch isoliert ist, dass bei einer Abkühlung im Eichraum durch rechtzeitigen Wasserdampfausgleich der Taupunkt nicht erreicht wird.

17. Vorrichtung zur Durchführung des Verfahrens nach Anspruch 4, gekennzeichnet durch einen Eichraum zur Aufnahme wasserdampfgesättigten Partialgases und durch eine Einrichtung zur Erzeugung eines gegenüber dem Partialgas erhöhten Temperaturniveaus an der Messstelle.

18. Vorrichtung nach Anspruch 17, gekennzeichnet durch eine Einrichtung zur Beheizung des Sensors nahe der Messstelle.

19. Vorrichtung nach Anspruch 17, dadurch gekennzeichnet, dass die Eichraumwandung durch Verdunstung gekühlt ist.

20. Vorrichtung nach Anspruch 14, dadurch gekennzeichnet, dass die Verbindung des Eichraumes (2") zum Wasserdampfreservoir (1") durch Verschlussbolzen (9") unterbrochen wird, der beim Einführen in den Eichraum eventuell in diesem vorhandenes Wasser durch eine Abflussbohrung hinausdrückt.

**Claims**

1. A process of calibrating sensors which measure a gas partial pressure and especially an oxygen partial pressure, wherein the sensor is exposed to the partial gas to be measured having a known partial pressure and a steam content which lies at the saturation limit due to a partial pressure gradient between steam-saturated gas and undersaturated gas, and the sensor signal is measured, characterized in that a defined flow of steam from steam-saturated to undersaturated gas is produced in a calibrating vessel and the location of the measuring point within this steam flow is fixed such that the steam partial pressure at the measuring point approaches but does not reach or exceed steam saturation.

2. The process as claimed in claim 1, characterized in that the steam flow is directed through narrow connections from a steam reservoir to the measuring point and from the latter to the undersaturated gas, the connection from the measuring point to the steam reservoir being greater than the connection of the measuring point to the undersaturated gas.

3. The process as claimed in claim 1 or 2, characterized in that the undersaturated gas is ambient air.

4. A process of calibrating sensors which measure a gas partial pressure and especially an oxygen partial pressure, wherein the sensor is exposed to the partial gas to be measured having a known partial pressure and a defined steam content and the sensor signal is measured, characterized in that steam saturation is adjusted in the partial gas to be measured and the sensor is kept at an elevated temperature with respect to the partial gas.

5. The process as claimed in claim 4, characterized in that the sensor is heated.

6. The process as claimed in claim 4, characterized in that by evaporation the partial gas is kept at a lower temperature level with respect to the sensor.

7. An apparatus for carrying out the method according to claim 1, characterized by a calibrating space (2, 2',2") connected, on the one hand, to a steam reservoir (1, 1',1") containing steam-saturated partial gas and, on the other hand, to an evasion reservoir (3) containing non-steam-saturated partial gas.

8. The apparatus as claimed in claim 7, characterized in that the connection of the calibrating space (2', 2") to the steam reservoir (1', 1") is greater than to the evasion reservoir.

9. The apparatus as claimed in claim 7 or 8, characterized in that the steam reservoir (1', 1") is a chamber filled partly with water (6', 6") and the evasion reservoir containing non-steam-saturated partial gas is constituted by the ambient air.

10. The apparatus as claimed in claim 9, characterized in that the calibrating space (2',2") and the steam reservoir (1',1") are embodied by two tubes closed at the bottom and at the top and placed concentrically one inside the other, the inner tube presenting the calibrating space and having an opening (5',5") for the tight introduction of the sensor to be calibrated, and in that the connections of the calibrating space with the steam reservoir, on the one hand, and with ambient air, on the other hand, are realized by bores (7',7",8',8") of suitable diameter.

11. The apparatus as claimed in claim 7 or 8, characterized in that the steam reservoir is embodied by an absorbent material which is impregnated with water.

12. The apparatus as claimed in one of claims 7 to 11, characterized in that the ratio between the passage cross sections in mm² of the connections (7',7",8',8") of the calibrating space (2',2") with the steam reservoir (1',1") and with the evasion reservoir and the volume of the calibrating space in mm³ is not less than 1:300.

13. The apparatus as claimed in claim 12, characterized in that said ratio is 1:1200 or higher.

14. The apparatus as claimed in one of claims 7 to 13, characterized in that the connection of the calibrating space (2") with the steam reservoir (1") is adapted to be closed during intervals between calibrations.

15. The apparatus as claimed in one of claims 7 to 14, characterized in that the connection of the calibrating space with the steam reservoir is covered by a porous diaphragm which is permeable to steam but impermeable to water.

16. The apparatus as claimed in one of claims 7 to 15, characterized in that the calibrating space (2, 2',2") is sufficiently insulated thermally so that upon cooling in the calibrating space the dew point is not reached because of a steam balance obtained in time.

17. An apparatus for carrying out the method according to claim 4, characterized by a calibrating space for receiving steam-saturated partial gas and by a means for producing an elevated temperature level with respect to the partial gas at the measuring point.

18. The apparatus as claimed in claim 17, characterized by a means for heating the sensor near the measuring point.

19. The apparatus as claimed in claim 17, characterized in that the calibrating space walls are cooled by evaporation.

20. The apparatus as claimed in claim 14, characterized in that the connection of the calibrating space (2") with the steam reservoir (1") is interrupted by a closing bolt (9") which, when introduced into the calibrating space, presses out any water present through a drain bore.

**Revendications**

1. Procédé d'étalonnage de capteurs qui mesurent une pression partielle de gaz et en particulier une pression partielle d'oxygène, dans lequel on expose le capteur au gaz partiel à mesurer, avec une pression partielle connue et avec une teneur en vapeur d'eau qui, en vertu d'une chute de pression partielle entre le gaz saturé de vapeur d'eau et le gaz sous-saturé, se situe à la limite de saturation et on mesure le signal de capteur, caractérisé en ce que dans un récipient d'étalonage on engendre un courant défini de vapeur d'eau, du gaz saturé de vapeur d'eau au gaz sous-saturé, et en ce que l'on fixe l'emplacement du point de mesure au sein de ce courant de vapeur d'eau de telle sorte que la pression partielle de vapeur d'eau au point de mesure approche de la saturation de vapeur d'eau mais n'atteint pas et ne dépasse pas celle-ci.

2. Procédé selon la revendication 1, caractérisé en ce que l'on conduit le courant de vapeur d'eau, en passant par des communications étroites, d'un réservoir à vapeur d'eau au point de mesure et de celui-ci au gaz sous-saturé, la communication du point de mesure au réservoir à vapeur d'eau étant plus grande que la communication du point de mesure au gaz sous-saturé.

3. Procédé selon l'une des revendications 1 et 2, caractérisé en ce que le gaz sous-saturé est de l'air ambiant.

4. Procédé d'étalonnage de capteurs qui mesurent une pression partielle de gaz et en particulier une pression partielle d'oxygène, dans lequel on expose le capteur au gaz partiel à mesurer, avec une pression partielle connue et avec une teneur déterminée en vapeur d'eau et on mesure le signal de capteur, caractérisé en ce que dans le gaz partiel à mesurer, on établit la saturation de vapeur d'eau, et en ce que l'on maintient le capteur à une température élevée relativement au gaz partiel.

5. Procédé selon la revendication 4, caractérisé en ce que l'on chauffe le capteur.

6. Procédé selon la revendication 4, caractérisé en ce que l'on maintient le gaz partiel, par vaporisation à un niveau de température inférieur à celui du capteur.

7. Dispositif pour la mise en œuvre du procédé selon la revendication 1, caractérisé par une chambre d'étalonnage (2, 2', 2") qui est reliée, d'une part, à un réservoir à vapeur d'eau (1, 1', 1") qui contient du gaz partiel saturé de vapeur d'eau et, d'autre part, à un réservoir d'échappement (3) qui contient du gaz partiel non saturé de vapeur d'eau.

8. Dispositif selon la revendication 7, caractérisé en ce que la communication de la chambre d'étalonnage (2', 2") au réservoir à vapeur d'eau (1', 1") est plus grande que celle de la chambre d'étalonnage au réservoir d'échappement.

9. Dispositif selon l'une des revendications 7 et 8, caractérisé en ce que le réservoir à vapeur d'eau (1', 1") est une chambre partiellement remplie d'eau (6', 6"), et en ce que le réservoir d'échappement, qui contient du gaz partiel non saturé de vapeur d'eau, est formé par l'air ambiant.

10. Dispositif selon la revendication 9, caractérisé en ce que la chambre d'étalonnage (2', 2") et le réservoir à vapeur d'eau (1', 1") sont formés par deux tubes fermés en haut et en bas, insérés concentriquement l'un dans l'autre, le tube intérieur formant la chambre d'étalonnage et présentant une ouverture (5', 5") pour l'introduction étanche du capteur à étalonner, et en ce que les communications de la chambre d'étalonnage au réservoir à vapeur d'eau, d'une part, et à l'air ambiant, d'autre part, sont assurées par des perforations (7', 7", 8', 8") de diamètre approprié.

11. Dispositif selon l'une des revendications 7 et 8, caractérisé en ce que le réservoir à vapeur d'eau est formé par une matière absorbante imbibée d'eau.

12. Dispositif selon l'une des revendications 7 à 11, caractérisé en ce que le rapport entre, d'une part, les sections de passage, en mm$^2$, des communications (7', 7", 8', 8") de la chambre d'étalonnage (2', 2") au réservoir à vapeur d'eau (1', 1") et au réservoir d'échappement et, d'autre part, le volume de la chambre d'étalonnage en mm$^3$ n'est pas inférieur à 1:300.

13. Dispositif selon la revendication 12, caractérisé en ce que ce rapport est de 1:1200 ou plus grand.

14. Dispositif selon l'une des revendicatios 7 à 13, caractérisé en ce que la communication de la chambre d'étalonnage (2") au réservoir à vapeur d'eau (1") peut être fermée dans les pauses d'étalonnage.

15. Dispositif selon l'une des revendications 7 à 14, caractérisé en ce que la communication de la chambre d'étalonnage au réservoir à vapeur d'eau est recouverte par un diaphragme poreux perméable à la vapeur d'eau mais imperméable à l'eau.

16. Dispositif selon l'une des revendications 7 à 15, caractérisé en ce que la chambre d'étalonnage (2, 2', 2") est suffisamment isolée thermiquement pour qu'en cas de refroidissement dans la chambre d'étalonnage, par compensation de vapeur d'eau en temps voulu, le point de rosée ne soit pas atteint.

17. Dispositif pour la mise en œuvre du procédé selon la revendication 4, caractérisé par une chambre d'étalonnage pour recevoir du gaz partiel saturé de vapeur d'eau et par un dispositif pour engendrer au point de mesure un niveau de température plus élevé que celui du gaz partiel.

18. Dispositif selon la revendication 17, caractérisé par un dispositif pour le chauffage du capteur près du point de mesure.

19. Dispositif selon la revendication 17, caractérisé en ce que la paroi de la chambre d'étalonnage est refroidie par vaporisation.

20. Dispositif selon la revendication 14, caractérisé en ce que la communication de la chambre d'étalonnage (2") au réservoir à vapeur d'eau (1") est interrompue par un goujon de fermeture (9") qui, lorsqu'on l'introduit dans la chambre d'étalonnage, expulse par une perforation d'écoulement l'eau qui s'y trouve éventuellement.

## Fig. 1

$P_{H_2O}$ (1)

$P_{H_2O}$ (2)

$P_{H_2O}$ (3)

4

1

2

3

## Fig. 2

5'

8'

7'

2'

1'

6'

Fig.3